(19) Europäisches Patentamt
European Patent Office
Office européen des brevets

(11) **EP 2 039 372 A1**

(12) **EUROPEAN PATENT APPLICATION**

(43) Date of publication:
**25.03.2009 Bulletin 2009/13**

(51) Int Cl.:
***A61L 9/12*** (2006.01)

(21) Application number: **08253109.6**

(22) Date of filing: **23.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **24.09.2007 US 974669 P**

(71) Applicant: **McNeil-PPC, Inc.**
**Skillman, NJ 08558 (US)**

(72) Inventors:
• **Gannon, Elaine M.**
**Hoboken, NJ 07030 (US)**
• **Moscherosch, Michael H.**
**Doylestown, PA 19801 (US)**

(74) Representative: **Kirsch, Susan Edith**
**Carpmaels & Ransford**
**43-45 Bloomsbury Square**
**London WC1A 2RA (GB)**

(54) **Fragrance emitting patch and compact for holding a plurality of such patches**

(57)     A fragrance emitting patch kit including a compact having an internal compartment and a plurality of fragrance emitting patches positioned within the internal compartment of the compact.

Fig. 9

**Description**

**CROSS REFERENCE TO RELATED APPLICATIONS**

This application claims priority to Application No. 60/974,669 filed on September 24, 2007, the entire contents of which are incorporated by reference herein.

**FIELD OF THE INVENTION**

The present invention relates to a fragrance emitting patch that a user can attach to the body or an article of clothing, and more particularly to such a patch including at least one layer including a fragrance and an adhesive applied to the patch for selectively securing the patch to the body or an article of clothing. The present invention also relates to a compact for holding a plurality of such fragrance emitting patches in stacked configuration.

**BACKGROUND OF THE INVENTION**

Fragrance emitting devices are generally used to deliver a pleasing scent to the user. These devices have been used in the past to mask undesirable odors and can also be functionalized with an odor-controlling agent. The prior art discloses fragrance emitting patches that purport to deliver fragrance when a user attaches the patch onto their body or an article of clothing. These patches generally include one more or more layers of material, at least one of which is provided with a fragrance. Fragrance emitting patches generally include a positioning adhesive applied to an external surface of the patch for selectively adhering the patch onto the user's skin or article of clothing. Patches with multiple layers may also include a construction adhesive used to affix the layers of the patch to one another.

The inventors have discovered that many fragrances used in known fragrance emitting patches will migrate into the adhesive components of the patch and undesirably interact with the adhesive by altering its chemical composition. In particular, the inventors have discovered the aromatic components of many fragrances tend to plasticize the end blocks of standard hot melt adhesives. The inventors have discovered that this interaction causes the adhesives to perform poorly by reducing the cohesiveness and internal strength of standard construction and positioning adhesives. Specifically, the inventors have discovered that the interaction between the fragrance and positioning adhesive may cause the patch to detach from the surface to which it is applied and in a multilayer construction the interaction of the fragrance with the construction adhesive may cause the undesirable delamination of the layers of the patch.

The inventors have further discovered that in order for a fragrance emitting patch to provide the desired intensity of scent, and in order for the scent to last for a sufficient period of time during use, the fragrance must be applied to the relevant layer of the patch in a relatively high add on amount. However, the inventors have discovered that the use of a high add on amount of fragrance exacerbates the degradation of the adhesive described above.

In view of the foregoing, the present invention provides a fragrance emitting patch that has the ability to incorporate high levels of fragrance without sacrificing the functionality of the construction and positioning adhesives used within the patch.

According to another aspect of the invention, the present invention also relates to a compact for carrying a plurality of fragrance emitting patches in a stacked configuration. The compact permits a plurality of the patches to be carried in a convenient portable manner and also facilitates the easy removal of a patch from the compact.

**SUMMARY OF THE INVENTION**

In view of the foregoing, the present invention provides, according to a first aspect of the invention, a fragrance emitting patch kit including a compact having an internal compartment and
a plurality of fragrance emitting patches positioned within the internal compartment of the compact, the plurality of patches arranged in a stacked configuration, and wherein each of the patches includes a terminal peripheral edge, the plurality of patches being arranged such that the terminal peripheral edge of each patch includes a portion that is offset relative to a portion of the terminal peripheral edge of each directly adjacent patch in the stack.

**BRIEF DESCRIPTION OF THE DRAWINGS**

FIG. 1 is a schematic view of a fragrance emitting patch in accordance with the invention adhered to the surface of an undergarment;
FIG. 2 is a bottom plan view of a fragrance emitting patch in accordance with the invention with the removable backing layer thereof partially torn away to reveal the positioning adhesive thereunder;

FIG. 3 is a cross sectional view of the patch shown in FIG. 2 taken along line 3-3 thereof;

FIG. 4 is a top plan view of a fragrance emitting patch in accordance with another embodiment of the invention;

FIG. 5 is a cross sectional view of the patch shown in FIG. 4 taken along line 5-5 thereof;

FIG. 6 is a top plan view of a fragrance emitting patch in accordance with yet another embodiment of the invention;

FIG. 7 is a cross sectional view of the patch shown in FIG. 6 taken along line 7-7 thereof.

FIG. 8 is a perspective view of a fragrance emitting patch kit including a compact and a plurality of fragrance emitting patches arranged within the compact, the compact is depicted in an open configuration showing the plurality patches arranged therein;

FIG. 9 is a detailed view of the plurality of patches shown in Fig. 8, showing the stacked arrangement of the patches;

FIG. 10 is a perspective view of a package for holding the fragrance emitting patch kit according to the present invention; and

FIG. 11 is a perspective view of a package for holding a plurality of replacement patches for the fragrance emitting patch kit according to the present invention.

## DETAILED DESCRIPTION OF THE INVENTION

As used herein, the term "construction adhesive" refers to any adhesive that is used to join two layers of material to one another.

As used herein, the term "positioning adhesive" refers to any adhesive that is used to removably attach a fragrance emitting patch to a user's skin or clothing.

In one embodiment of the invention, the fragrance emitting patch according to the present invention is intended to be applied to a user's undergarment, such as a woman's panty, during use, to thereby provide a fresh scent to undergarment. The inventors have found that in order to achieve long lasting fragrance levels that can be detected from the perennial region of the user to the nose, a high amount of fragrance must be incorporated into the fragrance emitting patch. The inventors have found that users can detect a product including a fragrance applied to one of the layers of the patch in an add on amount of greater than 3 gsm ($g/m^2$). In one embodiment of the invention, a fragrance is applied to at least one of the layers of the fragrance emitting patch in an amount of between about 3 gsm and about 15 gsm.

The inventors have discovered that purposely selecting fragrances that are substantially insoluble in the adhesive compounds used in the fragrance emitting patch minimizes the undesirable reaction between these components. The Hildebrand solubility parameter is used often in chemistry to predict when two solutions are soluble in one another. According to the theory proposed by Dr. Joel Hildebrand, two solutions will be soluble when the Hildebrand solubility parameter is equal, and insoluble when the Hildebrand solubility parameter is not equal. The difference between the two values is roughly related to the extent of insolubility between the two solutions. The Hildebrand solubility parameter ($\delta(SI)$) is derived from the heat of vaporization ($\Delta H$), the universal gas constant (R), the temperature (T), and the molar volume of the solution ($V_m$), and is calculated using the following formula:

$$\delta(SI) = [(\Delta H - RT) / V_m]^{1/2}$$

The resulting value is a property of a particular solution at a given temperature. In the international system of units (SI), the universal gas constant (R) is approximately 8.314 $J \cdot K^{-1} \cdot mol^{-1}$. The Hildebrand solubility parameter has the units of $MPa^{1/2}$.

The Hildebrand solubility parameter of common adhesives and fragrances is provided in TABLE 1 below:

**TABLE 1**

| Classification | Solution | $\delta(SI)$ |
|---|---|---|
| Common Adhesives | Sytrenic Block Copolymers and Tackifying Resins | 14.4-18.6 |
| | Polyethylene, EVA | 17-18.6 |
| | Polypropylene Polymers | 17.2-19.2 |

(continued)

| Classification | Solution | $\delta$(SI) |
|---|---|---|
| Common Fragrances | Pine Oil | 17.6 |
| | d-Limonene | 16.5 |
| | Vanillin | 24.7 |
| | Eugenol | 22.2 |
| | Citral | 18.7 |
| | Carvone | 18.7 |
| | Jasmone | 18.4 |

According to the present invention, the adhesive(s) and fragrance(s) employed in the fragrance emitting patch have a solubility parameter absolute value difference of greater than 1.5, preferably greater than 3.0 and most preferably greater than 5.0. This relationship can be expressed by the follow equation:

$$| \delta_a - \delta_f | > 1.5;$$

where

$\delta_a$ = Hildebrand solubility parameter of the adhesive, and

$\delta_f$ = Hildebrand solubility parameter of the fragrance.

Selection of a fragrance(s) and an adhesive(s) 20 satisfy the above equation insures that fragrance does not adversely interact with the adhesive and thereby compromise the same. This insures that, even at high fragrance add on levels, the fragrance emitting patch will securely adhere to the surface to which it is applied and will not delaminate.

## DESCRIPTION OF THE PREFERRED EMBODIMENTS

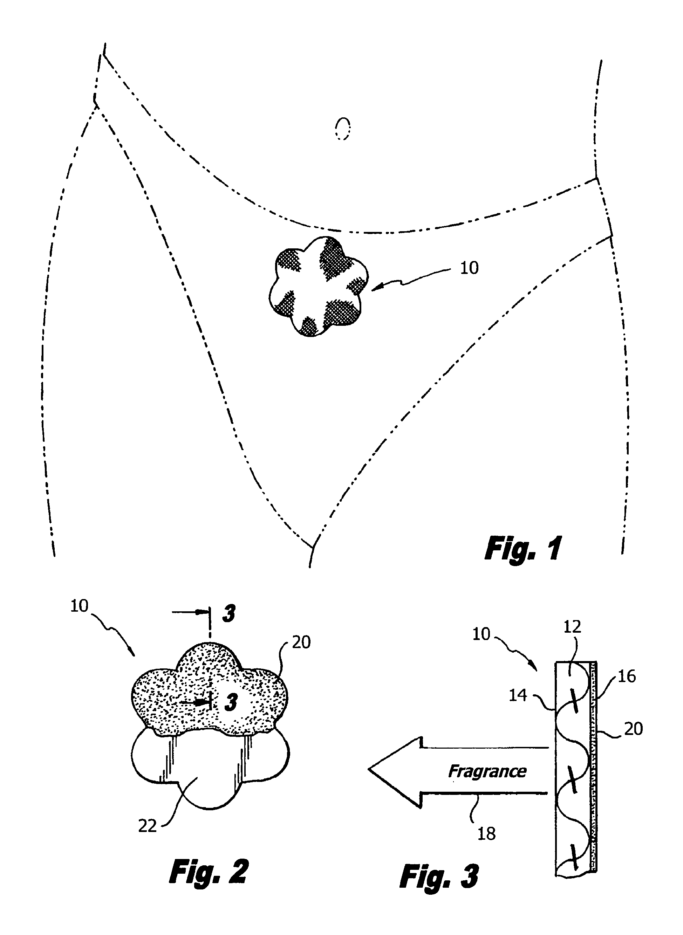

Fig. 1 illustrates an example of a fragrance emitting patch 10 according to the present invention, the patch 10 is adhered to the surface of a woman's undergarment to provide a fresh scent thereto. Referring to Figs. 2 and 3, the patch 10 includes, according to a first embodiment of the invention, a porous primary layer 12 having a top surface 14 and a bottom surface 16. The primary layer 12 is provided with a fragrance represented by the numeral 18. The fragrance 18 may be applied to a top surface 14 or bottom 16 surface of the primary layer 12 or it may be infused into the primary layer 12 itself. The patch 10 is further provided with a positioning adhesive 20 applied to the bottom surface 16 of the primary layer 12. The positioning adhesive 20 allows a user to selectively apply the patch to a garment of clothing such as a undergarment, or directly to the skin.

The fragrance emitting patch 10 may be optionally provided with a removable backing layer 22, shown in Fig. 2, that is intended to protect the the positioning adhesive 20 prior to use of the patch 10. The backing layer 22 may be constructed of a suitable paper and/or polymeric film material. The surface of the backing layer 22 in contact with the positioning adhesive 20 may be provided with a non-stick coating such as silicone to facilitate the removal of the backing layer 22 by the user prior to use.

According to one aspect of the invention, the primary layer 12 is constructed from a porous non-woven web material. The primary layer 12 may be composed of only one type of fiber, such as polyester or polypropylene or it may include a mixture of more than one fiber. The primary layer 12 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the primary layer 12 has a basis weight in the range of about 10 gsm to about 75 gsm. Bi-component fibers may be made up of a polyester layer and a polyethylene sheath. Using a fusible fabric increases the ease with which the primary layer 12 may be mounted to any underlying layer should such an underlying layer be employed. According to another aspect of the invention, the porous primary layer 12 is constructed from a microporous polymeric film material.

According to one apsect of the invention, the porous primary layer 12 is provided with the fragrance 18. The fragrance 18 may be selected from one of the fragrances set forth in Table 1 above or may be selected from other common fragrances known to those of skill in the art. The fragrance 18 may also constitute a complex fragrance, i.e. a fragrance including a mixture of a number of different fragrance components. Typically, the solubility parameter $\delta_f$ of

such complex fragrance mixtures may be obtained from the commercial manufacturer of such fragrances. The fragrance 18 is preferably provided on or in the primary layer 12 in an amount greater than about 3 gsm (g/m$^2$), preferably between about 3 gsm and about 15 gsm.

According to one aspect of the invention, the bottom surface 16 of the porous primary layer 12 is provided with a positioning adhesive 20. Preferably the positioning adhesive 20 is applied to the bottom surface 16 in an amount between about 8 gsm to about 25 gsm. Suitable positioning adhesive 20 compositions include hot melt adhesives based on block copolymers such as linear or radial co-polymer structures having the formula (A-B)$_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

According to the present invention, the positioning adhesive 20 and the fragrance 18 should be selected such that they have a solubility parameter absolute value difference of greater than 1.5, preferably greater than 3.0 and most preferably greater than 5.0. This relationship can be expressed by the follow equation:

$$|\,\delta_{pa} - \delta_f\,| > 1.5;$$

where

$\delta_{pa}$ = Hildebrand solubility parameter of the positioing adhesive, and

$\delta_f$ = Hildebrand solubility parameter of the fragrance.

Selection of a fragrance 18 and a positioning adhesive 20 that satisfy the above equation insures that fragrance 18 does not adversely interact with the positioning adhesive 20 and thereby compromise the same. This insures that, even at high fragrance add on levels, the fragrance emitting patch will securely adhere to the surface to which it is applied.

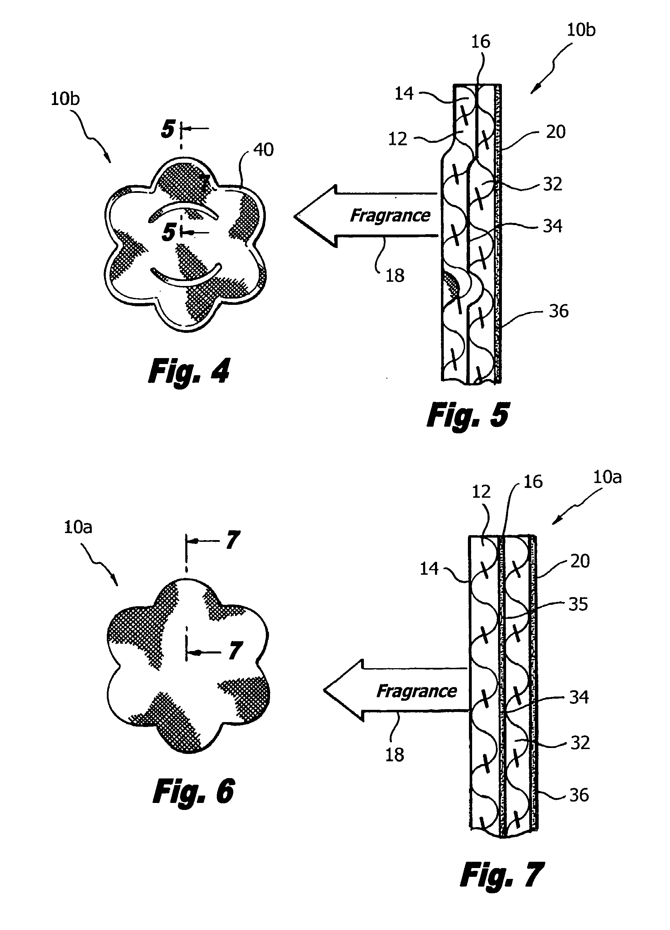

Referring to Figs. 6 and 7, a fragrance emitting patch 10a includes according to another embodiment of the invention, a porous primary layer 12 having a top surface 14 and a bottom surface 16. The primary layer 12 is provided with a fragrance represented by the numeral 18. The fragrance 18 may be applied to a top surface 14 or bottom 16 surface of the primary layer 12 or it may be infused into the primary layer 12 itself. The patch 10 further includes a secondary layer 32 having a top surface 34 and bottom surface 36. The secondary layer 32 is adhered to a bottom surface 16 of the primary layer 12 by a construction adhesive 35 that is arranged between the layers 12 and 32. The bottom surface 36 of the secondary layer 32 is provided with the positioning adhesive 20 that permits a user to selectively apply the patch 10a to a garment of clothing such as a undergarment, or directly to the skin.

The construction adhesive 35 is preferably selected from the same group of adhesives as the positioning adhesive 20. Thus suitable construction adhesive 35 compositions include hot melt adhesives based on block copolymers such as linear or radial co-polymer structures having the formula (A-B)$_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

As shown in Fig. 7, the construction adhesive 35 is preferably applied to a bottom surface 16 of the primary layer 12 in an amount between 1 gsm and 25 gsm. According to an aspect of the invention, the construction adhesive 35 and the fragrance 18 are applied in an amount such that a ratio of the amount of construction adhesive 35 employed in the patch 10a to amount of fragrance 18 incorporated into the patch 10a is between about 0.333 to about 1.67. This relationship can be expressed by the following equation:

$$1.67 > A_{ca} / A_f > 0.333,$$

where
$A_{ca}$ = Add on amount of construction adhesive, and
$A_f$ = Add on amount of fragrance.

According to the present invention, the construction adhesive 35 and the fragrance 18 should be selected such that they have a solubility parameter absolute value difference of greater than 1.5, preferably greater than 3.0 and most preferably greater than 5.0. This relationship can be expressed by the follow equation:

$$|\delta_{ca} - \delta_f| > 1.5;$$

where
$\delta_{ca}$ = Hildebrand solubility parameter of the construction adhesive, and
$\delta_f$ = Hildebrand solubility parameter of the fragrance.
Selection of a fragrance 18 and a construction adhesive 35 that satisfy the above equation insures that fragrance 18 does not adversely interact with the construction adhesive 35 and thereby compromise the same. This insures that, even at high fragrance add on levels, the layers of the fragrance emitting patch 10a will remain secured adhered to one another and will not delaminate.

According to one aspect of the invention, the secondary layer 32 may be a non-porous layer. In one preferred embodiment of the invention, the secondary layer 32 is a non-porous polymeric film such as polyethylene or polypropylene film.

Alternatively, the secondary layer 32 may be a porous layer. A porous secondary layer 32 may be a nonwoven material composed of only one type of fiber, such as polyester or polypropylene or it may include a mixture of more than one fiber. The secondary layer 32 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Bi-component fibers may be made up of a polyester layer and a polyethylene sheath. Using a fusible fabric increases the ease with which the secondary layer 32 may be mounted to an adjacent layer, e.g. the primary layer 12. According to another aspect of the invention, the secondary layer 32 is constructed from a microporous polymeric film material.

Referring to Figs. 4 and 5, a patch 10b includes according to a yet another embodiment of the invention, a porous primary layer 12 having a top surface 14 and a bottom surface 16. The primary layer 12 is provided with a fragrance represented by the numeral 18. The fragrance 18 may be applied to a top 14 or bottom 16 surface of the primary layer 12 or it may be infused into the primary layer 12 itself. The patch 10b further includes a secondary layer 32 having a top 34 and bottom surface 36. The bottom surface 36 of the secondary layer 32 is provided with the positioning adhesive 20 that permits a user to selectively apply the patch to a garment of clothing such as an undergarment or directly to the skin.

In the embodiment of the invention shown in Figs. 4 and 5, the primary layer 12 is secured to the secondary layer 32 in an adhesive free manner. For example, the primary layer 12 may be secured to the secondary layer by embossing using heat and pressure to fuse the primary layer 12 to the secondary layer 32. The primary layer 12 and secondary layer 32 may be constructed from nonwoven materials including heat fusible fibers to facilitate the bonding of the layers 12 and 32. In the embodiment shown in Fig. 4 and 5, the patch 10c includes a bonded area 40 around the periphery of the patch 10b.

The patches 10, 10a and 10b described herein preferably have a thickness in the range of between about 0.25 mm and about 2.0 mm.

## Example #1

A fragrance emitting patch according to the present invention may be constructed to include a 30 gsm primary layer made from a spunlace nonwoven material and a secondary non-porous layer made from a 10 gsm polyethylene film. A sytrenic block copolymer construction adhesive having a solubility parameter $\delta_{ca}$ of 14.8 is applied to a bottom surface of the primary layer in an amount of 5 gsm to adhere the primary layer to the secondary layer. The bottom surface of the secondary layer is provided with a sytrenic block copolymers positioning adhesive having a solubility parameter $\delta_{pa}$ of 15. A fragrance consisting of Eugenol having a solubility parameter $\delta_f$ of 22.2 is applied to a top surface of the primary layer in an amount of 10 gsm. In this example, the absolute value difference of the solubility parameter of the construction adheisve and the fragrance is 7.4 and the absolute value difference of the solubility parameter of the positioning adhesive and the fragrance is 7.2. A ratio of the add on amount of construction adhesive relative to the add on amount of fragrance is 0.5.

**Example #2**

Another fragrance emitting patch in accordance with the invention may be constructed in identical fashion to the patch described in Example #1 except that the secondary non-porous layer of example 1 is replaced with a 30 gsm layer spunlace nonwoven material.

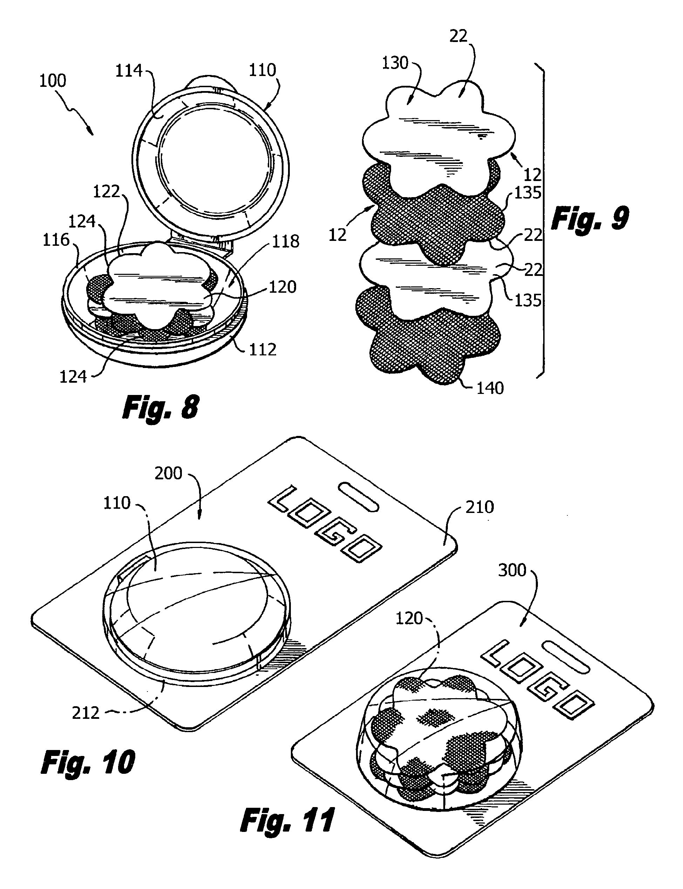

Referring to Fig. 8, the present invention also provides a fragrance emitting patch kit generally identified by the numeral 100. The kit 100 includes a compact 110 and a plurality of fragrance emitting patches 120 of the type described above. The compact 110, according to one embodiment of the invention, includes a base 112 and a cover 114 that is hingedly mounted to the base 112. The base 112 is mounted to the cover 114 such that a user may manually alternate between an open configuration and a closed configuration. In the embodiment shown in Fig. 8, the compact 110 includes a circular vertically extending wall 116. The circular vertically extending wall 116 functions to define an internal compartment 118 for holding the plurality of patches 120. As shown, the plurality of patches 120 are arranged in a stacked configuration, such that one patch overlies an adjacent patch. The compact 110, and compartment 118, are preferably constructed to hold from about 10 to about 100 patches, more preferably between about 20 and 60.

Referring to Figs. 8 and 9, according to one embodiment of the fragrance emitting patch kit 100 according to the present invention, the plurality of fragrance emitting patches 120 are arranged within the internal compartment 118 of the compact 110 in an "offset" or "non-aligned" stacked configuration. Specifically, each of the patches 120 includes a terminal peripheral edge 122 and each patch is arranged such that at least a portion 124 of the terminal peripheral edge is offset relative to a portion 126 of the terminal peripheral edge of each directly adjacent patch 120 in the stack. Stated another way, the patches 120 are arranged in a stacked configuration such that the terminal peripheral edge 122 of each patch 120 includes a least a portion 124 that is not aligned with the terminal peripheral edge 122 of each adjacent patch. This "offset" or "non-aligned" stacked configuration patches 120 facilitates the easy removal of each patch 120 from the stack of underlying patches. Preferably the portion 124 of the terminal peripheral edge 122 of each patch that is offset relative to the portion of a terminal peripheral edge 122 of each directly adjacent patch in said stack is offset relative to said portion by distance of from about 2 mm to about 15 mm.

Referring to Fig. 9, according to another embodiment of the fragrance emitting patch kit according to the present invention, the plurality of fragrance emitting patches 120 are arranged in a stacked configuration and include a top patch 130, a bottom patch 140, a plurality of patches 135 arranged between the top patch 130, and bottom patch 140. Each of the plurality of patches 135 arranged between the top patch 130 and bottom patch 140 are arranged such that the primary layer12 of each patch 135 is arranged in surface to surface contact with a primary layer 12 of an adjacent patch the removable backing layer 22 is arranged in surface to surface contact with the removable backing layer 22 of a second adjacent patch. Stated another way, the patches 120 are arranged in stacked configuration such that patches are arranged in an alternating arrangement, i.e. primary layer 12 face up, primary layer 12 face down, primary layer 12 face up, primary layer 12 face down, and so on. In this manner, the primary layer 12 of one patch is arranged in surface to surface contact with the primary layer 12 of an adjacent patch and the removable backing layer 22 of one patch is arranged in surface to surface contact with the removable backing layer 22 of an adjacent patch.

Fig. 10 shows a package 200 for holding the fragrance emitting patch kit according to the present invention. The package 200 contains a compact 110 including a plurality of fragrance emitting patches 120 (not shown) as described above. In one embodiment of the invention, the package comprises a base 210 and window material 212 secured to the base 210. The base 210 may be constructed from cardboard or other suitable packaging material. The window material 212 may be constructed from a suitable transparent material. The window material 212 encases the compact 110 but at the same time permits the consumer to view the compact 110 prior to purchase. After a user uses all of the patches 120 contained with the compact 110, the user may purchase additional patches 120 and refill the compact 110. Referring to Fig. 11, there is shown a package 300 for holding a plurality of replacement fragrance patches 120. As shown, the package 300 only contains a plurality of replacement fragrance patches 120, i.e. it does not contain an additional compact 110. In this manner, when a user uses all of the patches 120 contained within the compact 110, the user may purchase a package 300 with additional replacement patches 120. Preferably, the patches 120 contained within the package 300 are also arranged within a stacked configuration to permit the user to easily remove the patches 120 from the package 300 and simply place such patches 120 in the compact 110 for use.

**Claims**

1. A fragrance emitting patch kit comprising:

   a compact having an internal compartment; and
   a plurality of fragrance emitting patches positioned within the internal compartment of the compact, said plurality of patches arranged in a stacked configuration, and wherein each of said patches includes a terminal peripheral edge, said plurality of patches being arranged such that the terminal peripheral edge of each patch includes a portion that is offset relative to a portion of the terminal peripheral edge of each directly adjacent patch in said stack.

2. The kit according to claim 1, wherein the portion of the terminal peripheral edge of each patch that is offset relative to the portion of a terminal peripheral edge of each directly adjacent patch in said stack is offset relative to said portion by distance of from about 2 mm to about 15 mm.

3. The kit according to claim 1 or claim 2, wherein each of the fragrance emitting patches has a thickness in the range of between about 0.25 mm and about 2.0 mm.

4. The kit according to any preceding claim, wherein said plurality of patches are arranged in a vertical stacked configuration.

Fig. 1

Fig. 2

Fig. 3

10b

40

**Fig. 4**

16
14
12
10b
20
32
34
36
18

Fragrance

**Fig. 5**

10a

7

7

**Fig. 6**

12
16
10a
14
20
35
34
32
36
18

Fragrance

**Fig. 7**

Fig. 8

Fig. 9

Fig. 10

Fig. 11

**Europäisches Patentamt**
**European Patent Office**
**Office européen des brevets**

# EUROPEAN SEARCH REPORT

**Application Number**

EP 08 25 3109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 399 404 A (LAUGHLIN THOMAS J [US] ET AL) 21 March 1995 (1995-03-21)<br>* column 8, lines 25-27 *<br>* the whole document *<br>----- | 1-4 | INV.<br>A61L9/12 |
| Y | US 2006/251609 A1 (SOJKA MARCI E [US] ET AL) 9 November 2006 (2006-11-09)<br>* paragraphs [0007], [0049], [0056]; figures 3-5 *<br>----- | 1-4 | |
| Y | WO 98/24393 A (MINNESOTA MINING & MFG [US]) 11 June 1998 (1998-06-11)<br>* abstract; figures 2-5 *<br>* the whole document *<br>----- | 1-4 | |
| Y | WO 03/035510 A (3M INNOVATIVE PROPERTIES CO [US]; CAMERON BRIAN D [US]; CHANG SHU KUN) 1 May 2003 (2003-05-01)<br>* page 4, line 12 - page 5, line 10; figures *<br>----- | 1-4 | |
| A | CA 290 761 A (WATSON HENRY P) 25 June 1929 (1929-06-25)<br>* the whole document *<br>----- | 1-4 | **TECHNICAL FIELDS SEARCHED (IPC)**<br><br>A61L |

The present search report has been drawn up for all claims

| Place of search | Date of completion of the search | Examiner |
|---|---|---|
| Munich | 19 January 2009 | Nissen, Vagn |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

**ANNEX TO THE EUROPEAN SEARCH REPORT
ON EUROPEAN PATENT APPLICATION NO.**

EP 08 25 3109

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2009

| Patent document cited in search report | | Publication date | Patent family member(s) | | Publication date |
|---|---|---|---|---|---|
| US 5399404 | A | 21-03-1995 | NONE | | |
| US 2006251609 | A1 | 09-11-2006 | AU | 2006246414 A1 | 16-11-2006 |
| | | | EP | 1901919 A1 | 26-03-2008 |
| | | | JP | 2008539867 T | 20-11-2008 |
| | | | KR | 20080012287 A | 11-02-2008 |
| | | | WO | 2006121599 A1 | 16-11-2006 |
| WO 9824393 | A | 11-06-1998 | AT | 238746 T | 15-05-2003 |
| | | | AU | 735536 B2 | 12-07-2001 |
| | | | AU | 5689098 A | 29-06-1998 |
| | | | CA | 2273921 A1 | 11-06-1998 |
| | | | CN | 1239418 A | 22-12-1999 |
| | | | DE | 69721569 D1 | 05-06-2003 |
| | | | DE | 69721569 T2 | 20-11-2003 |
| | | | EP | 0955983 A1 | 17-11-1999 |
| | | | HK | 1025731 A1 | 08-04-2004 |
| | | | JP | 2001505462 T | 24-04-2001 |
| | | | NO | 992639 A | 01-06-1999 |
| | | | NZ | 335561 A | 25-05-2001 |
| WO 03035510 | A | 01-05-2003 | AT | 364561 T | 15-07-2007 |
| | | | DE | 60220703 T2 | 07-02-2008 |
| | | | EP | 1448456 A1 | 25-08-2004 |
| | | | ES | 2286331 T3 | 01-12-2007 |
| | | | JP | 2005506885 T | 10-03-2005 |
| CA 290761 | A | 25-06-1929 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60974669 B